# EUROPEAN PATENT APPLICATION

(11) **EP 4 556 120 A1**
(43) Date of publication of application: **21.05.2025**
(21) Application number: 23383158.5
(22) Date of filing: 14.11.2023
(51) Int. Cl.: B01L 3/02

(54) **ENGINEERED PIPETTE TIPS FOR LIQUID AND SOLID SAMPLE HANDLING**

(71) Applicant: European Molecular Biology Laboratory, 69117 Heidelberg (DE)
(72) Inventor: BERNARDELLO, Matteo, 08014 Barcelona (ES); VIKAS, 08329 Teià (ES)
(74) Representative: Krauss, Jan

(57) **Abstract**

The present invention relates to a pipette tip for handling of liquids comprising at least one aperture at a distal end that is positioned in a way that objects positioned essentially in line with the longitudinal axis are not directly hit by the stream of liquid when handling liquids with the pipette tip. The present invention further relates to an assembly comprising the pipette tip according to the invention attached either directly or via an adapter piece to a micropipette, a method of treating a sample with a liquid in a way that objects positioned essentially in line with the longitudinal axis are not directly affected by the stream of liquid when handling liquids with the pipette tip according to the invention, a kit, and respective uses for handling liquids and for treating a sample with a liquid.

## Description

The present invention relates to a pipette tip for handling of liquids comprising at least one aperture at a distal end that is positioned in a way that objects positioned essentially in line with the longitudinal axis are not directly hit by the stream of liquid when handling liquids with the pipette tip. The present invention further relates to an assembly comprising the pipette tip according to the invention attached either directly or via an adapter piece to a micropipette, a method of treating a sample with a liquid in a way that objects positioned essentially in line with the longitudinal axis are not directly affected by the stream of liquid when handling liquids with the pipette tip according to the invention, a kit, and respective uses for handling liquids and for treating a sample with a liquid.

### Background of the invention

Organoids are unique, emerging biological models for fundamental and applied research, holding great promise in drug screening and personalized medicine. In a typical tissue culture lab setting, these organoids are generated and experimented with in liquid or soft hydrogel suspensions and require periodic media exchanges or supplementation with growth factors. In order to avoid loss of samples during such experimental procedure (including but not limited to downstream stainings, harvesting for -omics studies, etc.) media flow from aspiration and dispensing streams from existing pipettes affect the samples and often lead to unwanted aspiration of micron-sized organoids or to their damage. Similar challenges exist for other 3D samples such as animal embryos.

This suboptimal pipetting is a major road-block for automation, especially for soft and solid samples in liquid solutions. The prevalent practice in the labs is therefore to have researchers perform manual aspiration and dispensing of the media with extra care to avoid aspirating and imaging the samples, visually checking the presence and positions of the samples within the container during the operations. The viability of samples at the end of procedures (that can often extend over multiple days) can be highly affected by the operator's experience and attention.

This manual intervention therefore limits the potential of the organoid systems that are often used in high-throughput experiments, to provide statistical meaning to the involved study. Thus, the field needs automation of those procedures, which would result in standardized protocols and reliable sample viability. Liquid handler devices exist in the market, but, using conventional pipette tips, they are designed to deal with liquid bio-samples, therefore not considering eventual solid 3D specimens within the media container, producing the above-mentioned issues. The absence of an easy-to-integrate device and methodology hinders the development of automated protocols for 3D samples, and therefore the whole sectors relying on high throughput research.

Organoids cover huge areas of interest in life science such as genetic screens, disease modelling, drug discovery against cancer and infective pathologies, clinical trials and personalized medicine. It is worth noting that in December 2022 the FDA Modernization Act 2.0 was approved by the U.S. government bodies. Through this act, animal testing is no longer required to assess drugs' safety and efficacy. EU's "Replacement, Reduction and Refinement" policy also encourages a transition away from animal samples. Therefore, in-vitro systems such as stem-cell derived organoids are in the front line as biological models for drug testing.

However, laboratories working with this novel specimen, both in academia and industry, still rely on tools that were designed for liquid bio-samples. The proposed design for novel pipette tips is therefore in exact timing with the market trends and needs for both industry and academia. Our invention will not only increase the efficiency in organoids' protocols performed manually but can also be integrated into automated protocols. In fact, through these designs, one could convert many of the already existing liquid-handler robots into 3D-sample-handler machines, with little effort. In turn, the automation of such protocols on organoids will boost the high-throughput studies in both academia and industry.

US9733169B2 discloses a pipette tip device for use in dispersive SPE. The device includes a pipette tip having a lower barrier, loose sorbent that is freely moveable during the extraction process, and a baffle system that is shaped to disrupt the flow of liquid sample that is aspirated into the pipette tip. The baffle system includes an insert that may be separate from or monolithic with the interior of the pipette tip.

US 2014-0377147A1 discloses a chromatography pipette tip having a first vessel and a second vessel which is open at two opposite ends and which, in each instance, has a bottom orifice via which a sample liquid can either be sucked in or expelled. The two vessels are fluidically and sealably connected to one another. A reaction matrix is arranged in one of the two vessels, through which reaction matrix the sample liquid is pushed by means of a pipettor to which the chromatography pipette tip is connected when used as intended. The sample liquid is drawn in and dispensed in opposite directions by the chromatography pipette tip and flows through the reaction matrix only in one direction.

WO 2015/156331 A1 provides an attachment for liquid injection that enables simple injection of liquid into an introduction opening by simply attaching a tip part for a pipette or a pipette tip when injecting a liquid into an introduction opening, and also can achieve excellent sealing performance and suppress leakage during liquid injection without danger of deformation or damage to the pipette or pipette tip.

WO 2020/127902A1 relates to a pipette tip extension attachable to a pipette tip. The pipette tip extension comprises a proximal end, a distal end, and an exterior wall extending between the proximal end and the distal end. The exterior wall has an outer side and an inner side and forms at the proximal end a reception aperture for inserting a pipette tip. The pipette tip extension further comprises a bottom at the distal end, an inner cavity enclosed by the inner side of the exterior wall and the bottom, one or more distance elements arranged at the inner side of the exterior wall and protruding into the inner cavity, and a coating for interacting with a fluid present in a fluid uptake area.

WO 2020/132394A1 also relates to a pipette tip extension attachable to a pipette tip comprising a proximal end, a distal end, and an exterior wall extending between the proximal end and the distal end. The exterior wall has an outer side and an inner side and forms at the proximal end a reception aperture for inserting a pipette tip, and at the distal end a dispense aperture. The pipette tip extension further comprises an inner cavity, a distance element connected to the inner side of the exterior wall, and a constriction element. The constriction element (12) may define an end stop (23) for a pipette tip (20) and is configured as a fluid-permeable sieve-like structure.

Given the high impact of organoids' research, companies have attempted to provide solutions to the above-mentioned challenges. However, most of the devices are specialized to the culturing steps, with little results toward a general way for 3D samples, such as organoids, handling. Existing solutions aim at modifying the culturing plate geometry or by automating a gentle media exchange, both of which require changes to experimental procedure.

3D samples like organoids need frequent media exchange. The use of current tools can lead to sample aspiration or damage, limiting all of throughput, repeatability, and the implementation of automated solutions.

It is an object of the present invention to provide an alternative solution for liquid handling in biological samples, such as a gentle media exchange in case of organoid samples, which is practicable also in the use of automated liquid handling systems. Other objects and advantages will readily become apparent for the person of skill from studying the following more detailed description and examples.

The above object of the present invention is solved as claimed in the appended claims.

In a first aspect thereof, the above object of the present invention is solved by a pipette tip (1) for handling of liquids, comprising a proximal end (p) and a distal end (d) and a longitudinal axis (l) extending therebetween, an aperture (2) at the proximal end positioned essentially concentric to the longitudinal axis (l) for connecting the tip to a pipette, wherein the distal end (d) of the tip is closed; and at least one aperture (3) close to the distal end,
characterized in that
the at least one aperture (3) at the distal end (d) is positioned in a way that objects positioned essentially in line with the longitudinal axis are not directly affected and/or impaired by the stream of liquid when handling liquids with the pipette tip.

Preferred is the pipette tip (1) according to the present invention, wherein the at least one aperture at the distal end (3) forms an angle between 45 and 180, preferably between 90 and 180 degrees with the longitudinal axis (l) and is preferably positioned in an essentially orthogonal arrangement.

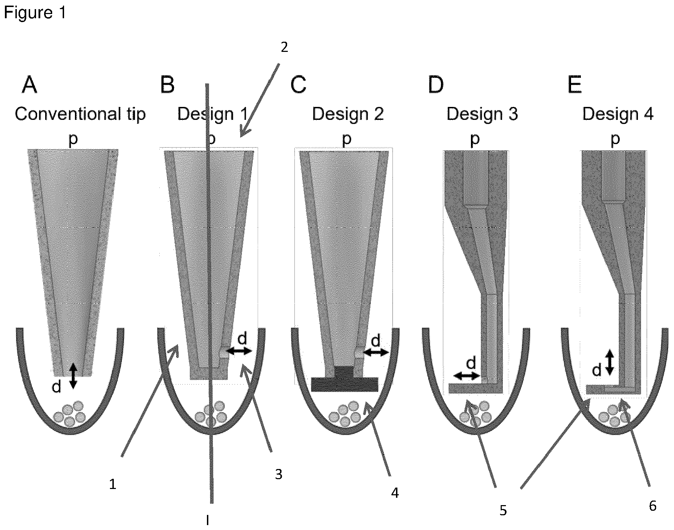

A conventional tip (shown in Fig. 1A) consists of a hollow cone with proximal and distal apertures (indicated respectively as `p' and 'd' in Fig 1A) that are concentric and aligned with a longitudinal axis (I). With this design, samples are directly affected and/or impaired by the flow of liquids handled, such as media. Therefore, the inventors in the context of the present invention have come up with a different design that is described here in the context of four different embodiments (designs) that can be manufactured and used in different ways. Of course, other variations are possible without deviation from the concept of the invention when considering the general disclosure of the invention as given herein. In general, the proposed device according to the invention decouples the two apertures' orientations. While the proximal aperture (2) is kept essentially concentric to the longitudinal axis (l), maintaining compatibility with the existing micropipettes, the distal aperture (3) forms an angle between about 45 and 180, preferably between about 90 and 180 degrees with that axis. During liquid, such as media, dispensing, the liquid is in some designs inserted into the container or vessel following the container's or vessel's walls, reducing the turbulence. This reduces potential damages to the samples, such as cells, cell pellets or organoids, and their movement, therefore reducing the time to wait between consecutive steps for their sedimentation. In addition, also while aspirating the flow does not interact directly with the sample(s). This reduced interaction drastically decreases the eventuality of aspirating (and losing) the sample(s).

The inventive design for the micropipette tips enables exchanging media without displacing the biological sample, thus minimizing samples' interactions to the aspiration and dispensing streams. The inventive design is fully compatible with existing instruments, machinery, and hardware in the laboratory. The inventive design is a low-cost device, readily integrable into existing lab procedures and allows automation of pipetting with soft and solid samples in liquid solutions with minimal exposure of the contained samples to the media flow. The inventive design therefore can minimize loss or destruction of samples, and can increase the efficiency, speed, and success rate of biological protocols. The inventive design finally also allows for and provides a gentle media exchange, in particular in automation of processes.

Preferred is the pipette tip (1) according to the present invention, wherein the distal end (d) of the tip is formed with the distal end (3) being closed, or the distal end is closed by a separate plug (4). Preferred is the pipette tip (1) according to the present invention, wherein the distal end (d) of the tip is formed with a ledge or protrusion (5) positioned essentially perpendicular to the longitudinal axis (I), preferably in an essentially round shape, and/or is further preferably provided off-centered from the longitudinal axis (l). The ledge or protrusion (5) may comprise a channel (6) for the flow of the liquids as handled. The size and shape of the ledge or protrusion (5) can furthermore be designed to impede the pipette tip from being inserted below a certain height within the sample container or vessel. In doing so, a trap for the samples is created by the ledge (5) and the container's or vessel's walls. Moreover, this permits to define a quantity of a liquid that may be left within the container during the aspiration step. These traps also enable knowing about the position of the samples within the container(s) without using additional tools, such as computer vision or microscopes.

In a second aspect thereof, the above object of the present invention is solved by an assembly, comprising the pipette tip (1) according to the present invention, attached either directly or via an adapter piece, such as a common pipette tip, to a micropipette, such as a handheld pipette or to a pipette of a liquid handling workstation, such as a positive displacement pipette, in particular an air displacement pipette.

In a third aspect thereof, the above object of the present invention is solved by a method of treating a sample with a liquid, the method comprising the following steps, i) providing the pipette tip (1) according to any one of claims 1 to 10, ii) attaching the pipette tip (1) either directly or via an adapter piece, such as a common pipette tip, to a micropipette, iii) a) aspirating a liquid into the pipette tip (1), and iv) a) dispensing the liquid from the pipette tip (1) into a suitable culture vessel comprising the sample, or iii) b) aspirating a liquid into the pipette tip (1) from the culture vessel comprising the sample, and iv) b) dispensing the liquid from the pipette tip (1) into a suitable vessel or container, in a way that objects positioned essentially in line with the longitudinal axis are not directly affected and/or impaired by the stream of liquid when handling liquids with the pipette tip (1).

In a fourth aspect thereof, the above object of the present invention is solved by the use of the pipette tip (1) according to the present invention for treating a sample with a liquid in a way that objects positioned essentially in line with the longitudinal axis are not directly hit by the stream of liquid when handling liquids with the pipette tip (1).

In a fifth aspect thereof, the above object of the present invention is solved by a kit for treating a sample with a liquid, comprising materials for performing a method according to the present invention, such as comprising a liquid selected from a buffer or cell culture medium for cultivating and/or maintaining and/or storing one or more samples, in particular for 3D organoid culture, tissue culture, preserving organs or parts thereof, a reagent for treating the sample, preferably for performing a cell lysis reaction, a staining reaction, a binding reaction, or for removing an embedding medium and/or a transport liquid for collecting particles for further analysis, and further comprising the pipette tip (1) and/or assembly according to the present invention.

As mentioned above, the above object of the present invention is solved by a specific pipette tip (1) for handling of liquids, such as cell culture media. The pipette tip (1) has a generally elongated body, preferably essentially cone-shaped, comprising a proximal end (p) on the side of the connection to, for example, a positive displacement pipette or a vacuum producing device, like an air displacement pump, like a micropipette with a plunger, and a distal end (d) on the side of the vessel to be emptied, stirred or filled. A longitudinal axis (l) extends between the proximal end (p) and the distal end (d).

An aperture (2) at the proximal end is positioned essentially concentric to the longitudinal axis (l) for connecting the tip to the vacuum producing device, e.g., a pipette. The distal end (d) of the tip is closed. Closure of the distal end may be achieved in any suitable way, for example the distal end (d) of the tip is formed with the distal end (3) being closed during production of the tip. Otherwise, the distal end may be closed by a separate plug (4).

The tip comprises at least one other aperture (3) close to the distal end. Importantly, the at least one aperture (3) at the distal end (d) is characterized in that it is positioned in a way that objects positioned essentially in line with the longitudinal axis are not directly affected and/or impaired by the stream of liquid when handling liquids with the pipette tip. The arrangement of the at least one aperture (3) at the distal end (d) causes that during liquid dispensing and/or aspiration/removal, the liquid is by design inserted into or removed from the container or vessel reducing turbulence. This reduces potential damages to the samples, such as cells, cell pellets or organoids, and their movement, therefore reducing the time to wait between consecutive steps for their sedimentation. The inventive design allows automation of pipetting with soft and/or solid samples in liquid solutions with minimal exposure of the contained samples to the media flow.

Therefore, preferred is the pipette tip (1) according to the present invention, wherein the at least one aperture at the distal end (3) forms an angle with the longitudinal axis (l) that is selected in in a way that objects positioned essentially in line with the longitudinal axis are not directly affected and/or impaired by the stream of liquid when handling liquids with the pipette tip. This may be at least of between about 20 and about 180 degrees, at least of between about 45 and about 180 degrees, preferably between about 90 and about 180 degrees with the longitudinal axis (l) and is preferably positioned in an essentially orthogonal arrangement. As mentioned above, more preferably, the at least one aperture at the distal end (3) is positioned in a manner that the stream of liquid as inserted or aspirated through the tip into the container or vessel follows the containers or vessel's walls.

In an embodiment of the pipette tip (1) according to the present invention, there are provided 1, 2, 3, 4, 5 or 6 apertures (3) at the distal end (d), for example opposite to each other (2 or 4 apertures), in a third of the circumference (3 apertures) or a pentagram (5 apertures) or star shaped (6 or more apertures).

In an embodiment of the pipette tip (1) according to the present invention, the distal end (d) of the tip is formed with a ledge or protrusion (5) positioned essentially perpendicular to the longitudinal axis (I), preferably in an essentially round shape, and/or is further preferably provided off-centered from the longitudinal axis (l). Preferably the ledge or protrusion has a in an essentially round shape, ellipsoid or a half circle shape, which may also be provided off-centered from the longitudinal axis (I), i.e. is extending more to one side than the other. The design of the ledge will follow the considerations of how to avoid turbulence in the given shape of a vessel or container when applying the medium.

Adding a physical barrier between distal aperture and sample further decreases the interactions between medium and sample, such as organoids. The barrier serves as a physical support for a distal aperture's channel directed upward. The barrier serves as a trap for the sample. The size and shape of the ledge or protrusion (5) can furthermore be designed to impede the pipette tip from being inserted below a certain height within the sample container or vessel. In doing so, a trap for the samples is created by the ledge (5) and the container's or vessel's walls. Moreover, this permits to define a quantity of a liquid that may be left within the container during the aspiration step. These traps also enable knowing about the position of the samples within the container(s) without using additional tools, such as computer vision or microscopes. This all avoids the need for visual inspection, easing the requirements for implementing the approach in automated systems.

In yet another embodiment of the pipette tip (1) according to the present invention, the ledge or protrusion (5) comprises at least one channel (6) for the flow of the liquids as handled. In this embodiment, the flow of the medium is preferably at an angle of between about more than 90 and about 180 degrees with respect to the longitudinal axis (I), i.e. "upwards" in the direction of the proximal end (p).

In a particularly preferred embodiment of the pipette tip (1) according to the present invention, "design 1" (see Figure 1B), the pipette tip (1) is sealed at the lower end and the distal aperture (3) faces the side of the tip, so that the media flow does not directly hit or aspirate the sample/specimen. This described design can be preferably implemented as either ready-to-use manufactured pipette tips with the new design or through a bench-top machine (similar to glass needle pulling devices e.g. Sutter instruments) that can be used in the lab to convert existing tips into this new design, closing the bottom aperture and drilling the distal aperture on the side.

In a particularly preferred embodiment of the pipette tip (1) according to the present invention, "design 2" (see Figure 1C), similar to the previous design, the distal aperture (3) is in the side/facing the side of the tip. This embodiment is derived from a conventional tip by closing the bottom aperture with a "stopper" or plug (4). The lower part of the plug is a circular structure whose diameter can be designed to be equal to the diameter of the container at a chosen height, forming a ledge. Through this feature, the tip cannot enter below the chosen height from the container's bottom, isolating the volume affected by the media flows from the samples, and preventing their unwanted squeezing.

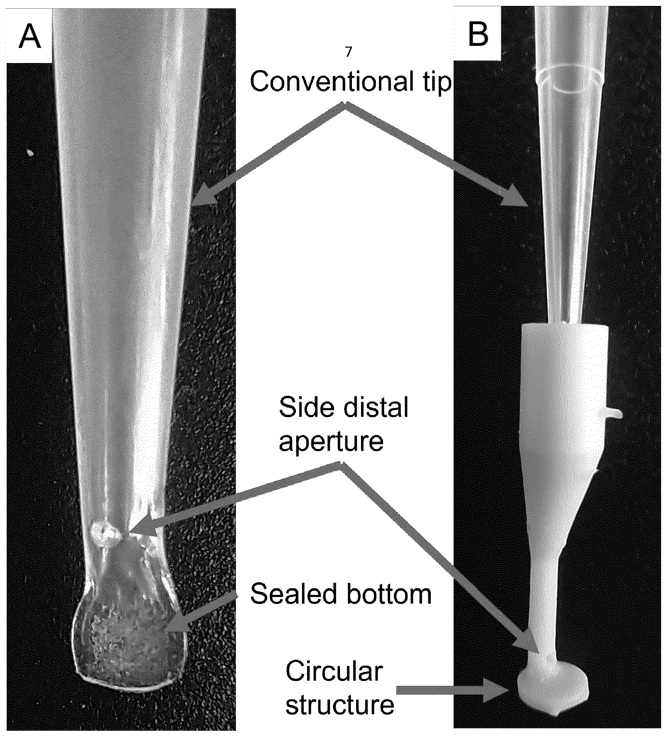

In a particularly preferred embodiment of the pipette tip (1) according to the present invention, "design 3" (see Figure 1D), the distal aperture (3) is directed toward the side of the tip. Here the circular structure on the bottom (the ledge) is integrated into the design. An example of a 3D printed prototype mounted as attachment to a conventional tip is shown in Fig. 2B.

In yet another particularly preferred embodiment of the pipette tip (1) according to the present invention, "design 4" (see Figure 1E), a u-shaped channel is crossing or is integrated into the bottom ledge circular structure, forming a distal aperture directed toward the upper part of the container. The aspiring and dispensing are therefore directed in the opposite ("upward") direction with respect to the samples' position, and the ledge/bottom structure still offers an additional protection for the specimen.

Although the invention is described here as a separate pipette tip, the concept of the invention can also be applied to other pipettes, such as a Pasteur-pipette, with a rubber bulb attached to the proximal end (p).

In general, the material of the pipette tip (1) according to the present invention can be any suitable material used for tips for liquid handling, e.g., suitable for cell culture. The pipette tip may be a disposable ("single use") pipette tip or a reusable pipette tip, such as a washable stainless-steel tip, glass or plastic, such as autoclavable PVC or PE.

All designs, and in particular designs 3 and 4 can be either manufactured as ready-to-use disposable pipette tips, or as attachments to conventional tips, or as washable stainless steel or glass tips. The choice of the actual implementation would depend on the need of the application (e.g., need for sterility).

The pipette tip (1) according to the present invention can generally be used and is compatible with existing pipettes, such as air displacement pipettes, in particular micropipettes. Therefore, the overall shape is essentially cone-shaped. Preferred is the pipette tip (1) according to the present invention, wherein the pipette tip is configured as being ejectable from a pipette tip by an ejection mechanism, for example of a handheld pipette or of a liquid handling workstation.

As mentioned above, the pipette tip (1) of the present invention is for the handling of liquids. The kind of liquid as such is not limited and depends on the intended purpose, preferably, the liquid is selected from a group comprising: a buffer or cell culture medium for cultivating and/or maintaining and/or storing one or more samples, in particular for 3D organoid culture, a reagent for treating the sample, preferably for performing a cell lysis reaction, a staining reaction, a binding reaction, or for removing an embedding medium a transport liquid for collecting particles for further analysis. More preferred is cell culture medium for cultivating and/or maintaining and/or storing one or more biological samples, in particular for cell culture or 3D organoid culture purposes.

With an important embodiment of the pipette tip (1) according to the present invention, the above object of the present invention is solved by a pipette tip that is connected to a micropipette either directly or via an adapter piece, such as a common pipette tip (7). This embodiment of the pipette tip (1) according to the present invention makes the tip even more compatible with existing technologies and can be used directly by friction-based fastening of the tip on a standard common pipette tip (7). Other adapters may be used as well but seem to be less convenient.

Another aspect of the present invention then relates to an assembly, comprising the pipette tip (1) according to the present invention, attached either directly or via an adapter piece, such as a common pipette tip, to a micropipette, such as a handheld pipette or to a pipette of a liquid handling workstation, in particular an air displacement pipette. This is another example for the compatibility of the pipette tip (1) according to the present invention.

Another aspect of the present invention then relates to a method of treating a sample with a liquid, the method comprising the following steps, i) providing the pipette tip (1) according to any one of claims 1 to 10, ii) attaching the pipette tip (1) either directly or via an adapter piece, such as a common pipette tip, to a micropipette, iii) a) aspirating a liquid into the pipette tip (1), and iv) a) dispensing the liquid from the pipette tip (1) into a suitable culture vessel or container comprising the sample, or iii) b) aspirating a liquid into the pipette tip (1) from the culture vessel or container comprising the sample, and iv) b) dispensing the liquid from the pipette tip (1) into a suitable vessel or container, in a way that objects positioned essentially in line with the longitudinal axis are not directly affected and/or impaired by the stream of liquid when handling liquids with the pipette tip (1).

Preferred is a method according to the present invention, wherein the step of aspirating is performed while the tip is moving into the direction of the bottom of the vessel or container ("descending"), and/or the step of dispensing is performed while the tip is moving into the direction of the top of the vessel or container ("ascending"). This provides a further decrease of any effect of the liquid on a sample.

As above, in the inventive method, the arrangement of the at least one aperture (3) at the distal end (d) causes that during liquid dispensing, the liquid is by design inserted into or aspirated from the container or vessel reducing turbulence. This reduces potential damages to the samples, such as cells, cell pellets or organoids, and their movement, therefore reducing the time to wait between consecutive steps for their sedimentation. The inventive design allows automation of pipetting with soft samples in liquid solutions with minimal exposure of the contained samples to the media flow.

With reference to what was described above, preferred is the method according to the present invention, wherein the culture vessel is a cell culture vessel, such as a 3D cell culture vessel, and the objects are selected from cells, such as embryonic cells, patient-derived tissues, organs or parts thereof, embryos or organoids.

The pipette tip (1) and/or assembly according to the present invention may be used with any tubes, centrifuge tubes, round bottom tubes or vials, centrifuge tubes, plates or box-shaped, containers, microtiter plates or vessels where a sample can be collected at, for example, the bottom thereof, and a liquid needs to be either added or removed.

Another aspect of the present invention then relates to the use of the pipette tip (1) according to the present invention for treating a sample with a liquid in a way that objects positioned essentially in line with the longitudinal axis are not directly hit by the stream of liquid when handling liquids with the pipette tip (1).

Another aspect of the present invention then relates to a kit for treating a sample with a liquid, comprising materials for performing a method according to the present invention, such as comprising a liquid selected from a buffer or cell culture medium for cultivating and/or maintaining and/or storing one or more samples, in particular for 3D organoid culture, tissue culture, preserving organs or parts thereof, a reagent for treating the sample, preferably for performing a cell lysis reaction, a staining reaction, a binding reaction, or for removing an embedding medium and/or a transport liquid for collecting particles for further analysis, and further comprising the pipette tip (1) and/or assembly according to the present invention. Another aspect of the present invention then relates to the use of this above kit for the handling of liquids, such as cell culture media, or treating a sample with a liquid according to the methods according to the invention.

Another aspect of the present invention then relates to the use of a pipette tip (1) according to the present invention for the handling of liquids, such as cell culture media, or for treating a sample with a liquid according to the methods according to the invention.

In the context of the present invention, the term "about" shall relate to a deviation from +/- 10% from a given value, unless otherwise indicated.

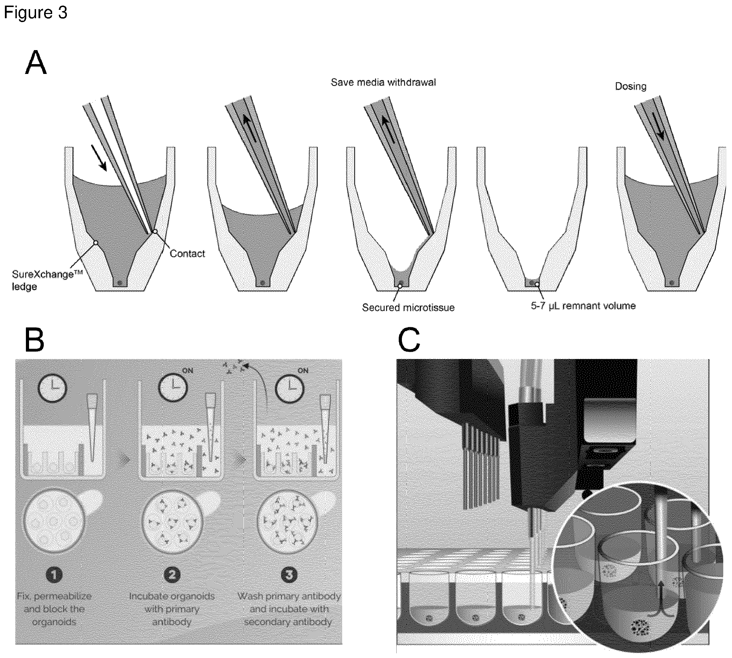

The working principles of pipette tips according to the art are shown in Fig. 3.

In Figure 3A, an Akura^{™} 96/384 Spheroid Microplate (InSphero AG) is shown. The product comprises a multiwell plate with specific geometry. The wells present oblique walls from which media is aspirated tilting the tip, and allegedly this assures that this prevents the aspiration of organoids. Advantageously, this is a simple solution, which allows for a possible integration into existing culturing protocols. Unfortunately, the technology focuses on the culturing step, and only comprises one spheroid per well. Expensive (50 to 100 Euro each single plate). In case spheroids need to be removed from the plate, no alternative handling strategy is provided.

In Figure 3B, the product Gri3D^{®} (SUN bioscience SA) is shown. The product comprises a multiwell plate with hydrogel forming microwells within a single well. Advantageously, this is a high-throughput device. The exchange of media is performed through a side well, avoiding sample contact. The product is expensive (500 Euro each single plate). In case spheroids need to be removed from the plate, no alternative handling strategy is provided. Also, the spheroid size depends on the plate used.

In Figure 3C, the product Automated Media Exchange module (Agilent Technologies, Inc.) is shown. Advantageously, this provides an automated module with washable vertical tips exchanging media on 96 U-bottom multiwell plates and automates tedious operations. The tips are simply positioned at the side of the well, and a slow exchange of the media avoids disturbing the spheroids. Nevertheless, only one spheroid per well can be handled. The device is specialized for culturing only. In case spheroids need to be removed from the plate, no alternative handling strategy is provided.

The embodiments according to the present invention eliminate the problem of the pipette tip's directional flow. This results in a general solution for cell, tissue, and/or organoid handling, independently from their stage, size, and quantity per well, and is easily adaptable in both manual and automated protocols.

The embodiments according to the present invention can be implemented with the same manufacturing process of conventional pipette tips, leading to a low-cost device, easily accessible from any lab, and compatible with existing micropipette standards and virtually any lab protocol.

The present invention relates to the following items:
Item 1. A pipette tip (1) for the handling of liquids, comprising a proximal end (p) and a distal end (d) and a longitudinal axis (l) extending therebetween, an aperture (2) at the proximal end positioned essentially concentric to the longitudinal axis (l) for connecting the tip to a pipette, wherein the distal end (d) of the tip is closed; and at least one aperture (3) close to the distal end,
   characterized in that
   the at least one aperture (3) at the distal end (d) is positioned in a way that objects positioned essentially in line with the longitudinal axis (l) are not directly hit by the stream of liquid when handling liquids with the pipette tip.
Item 2. The pipette tip (1) according to Item 1, wherein the at least one aperture at the distal end (3) forms an angle between 45 and 180 degrees, preferably between 90 and 180 degrees with the longitudinal axis (l) and is preferably positioned in an essentially orthogonal arrangement.
Item 3. The pipette tip (1) according to Item 1 or 2, wherein the distal end (d) of the tip is formed with the distal end (3) being closed, or the distal end is closed by a separate plug (4).
Item 4. The pipette tip (1) according to any one of Items 1 to 3, wherein the distal end (d) of the tip is formed with a ledge or protrusion (5) positioned essentially perpendicular to the longitudinal axis (l), preferably in an essentially round shape, which preferably creates a trap for the sample and/or is provided off-centered from the longitudinal axis (I).
Item 5. The pipette tip (1) according to Item 4, wherein the ledge or protrusion (5) comprises a channel (6) for the flow of the liquids as handled.
Item 6. The pipette tip (1) according to any one of Items 1 to 5, wherein the pipette tip is configured as being ejectable from a pipette tip by an ejection mechanism, for example of a handheld pipette or of a liquid handling workstation.
Item 7. The pipette tip (1) according to any one of Items 1 to 6, wherein the pipette tip is configured as maintaining compatibility with existing micropipettes, and preferably overall is essentially cone-shaped.
Item 8. The pipette tip (1) according to any one of Items 1 to 7, wherein the pipette tip is connected to a micropipette either directly or via an adapter piece, such as a common pipette tip (7).
Item 9. The pipette tip (1) according to any one of Items 1 to 8, wherein the pipette tip is disposable pipette tip or a reusable pipette tip, such as a washable stainless-steel tip.
Item 10. The pipette tip (1) according to any one of claims 1 to 9, wherein the liquid is selected from a group comprising: a buffer or cell culture medium for cultivating and/or maintaining and/or storing one or more samples, in particular for 3D organoid culture, a reagent for treating the sample, preferably for performing a cell lysis reaction, a staining reaction, a binding reaction, or for removing an embedding medium a transport liquid for collecting particles for further analysis.
Item 11. An assembly, comprising the pipette tip (1) according to any one of Items 1 to 10, attached either directly or via an adapter piece, such as a common pipette tip, to a micropipette, such as a handheld pipette or to a pipette of a liquid handling workstation, in particular an air displacement pipette.
Item 12. A method of treating a sample with a liquid, the method comprising the following steps, i) providing the pipette tip (1) according to any one of Items 1 to 10, ii) attaching the pipette tip (1) either directly or via an adapter piece, such as a common pipette tip, to a micropipette, iii) a) aspirating a liquid into the pipette tip (1), and iv) a) dispensing the liquid from the pipette tip (1) into a suitable culture vessel comprising the sample, or iii) b) aspirating a liquid into the pipette tip (1) from the culture vessel comprising the sample, and iv) b) dispensing the liquid from the pipette tip (1) into a suitable vessel or container, in a way that objects positioned essentially in line with the longitudinal axis are not directly affected and/or impaired by the stream of liquid when handling liquids with the pipette tip (1).
Item 13. The method according to Item 12, wherein the culture vessel is a cell culture vessel, such as a 3D cell culture vessel, and the objects are selected from cells or organoids.
Item 14. Use of the pipette tip (1) according to any one of Items 1 to 10 for treating a sample with a liquid in a way that objects positioned essentially in line with the longitudinal axis are not directly hit by the stream of liquid when handling liquids with the pipette tip (1).
Item 15. A kit for treating a sample with a liquid, wherein comprising a liquid selected from a group comprising: a buffer or cell culture medium for cultivating and/or maintaining and/or storing one or more samples, in particular for 3D organoid culture, and further comprising the pipette tip (1) according to any one of Items 1 to 10.

The invention will now be described further in the following examples with reference to the accompanying figures, nevertheless, without being limited thereto. For the purposes of the present invention, all references as cited are incorporated by reference in their entireties.

Figure 1 shows an overview of the conventional (A) and inventive (B-E) designs for pipette tips, all cross-section views. Pipette tips walls are in dark grey, while the channel through which the media flows is in light grey. 'p' and 'd' indicate respectively the proximal and distal apertures, while arrows indicate the direction of the aspirating and dispensing streams. Yellow spheres represent the sample under experimentation, while blue borders the container. Note that in conventional tips (A), the specimen is directly displaced/affected by the flow while in all the proposed designs (B-E), samples are not hit by the media streams.

Figure 2 shows in (A) Prototype for Design 1 (see figure 1) manufactured by sealing the bottom distal aperture of a conventional tip and drilling a new lateral aperture. (B) Prototype for Design 3 manufactured as attachment to a conventional pipette tip, through 3D printing. The attachment changes the conventional flow direction to the side and adds a circular structure as protection for the samples.

Figure 3 shows the working principle for the common (A) Akura^{™} 96 well Spheroid Microplate, (B) Gri3D^{®}, and (C) Automated Media Exchange module.

Figure 4 shows gastruloids used for the experiments on the first sample container, before (A) and after (B) 15x manual media exchanges using the engineered pipette tip according to the present invention.

Figure 5 shows gastruloids used for the present experiments, before (A) and after (B) 20x automatic media exchanges using the engineered pipette tip according to the present invention.

### Examples

### Engineered pipette tip as used according to the present invention

For both the following proof-of-concept experiments, the same devices were used. The sample containers were 1.5 ml centrifuge tubes. The engineered tip was 3D printed as an attachment to a conventional 1 ml pipette tip. The engineered tip decouples proximal and distal apertures in an orthogonal scheme, and it creates a barrier on the bottom (see Fig. 1). The same procedures were executed also with a conventional 1 ml pipette tip for comparison.

### Experiment 1: Manual medium exchange

### Experimental procedure with engineered pipette tips:

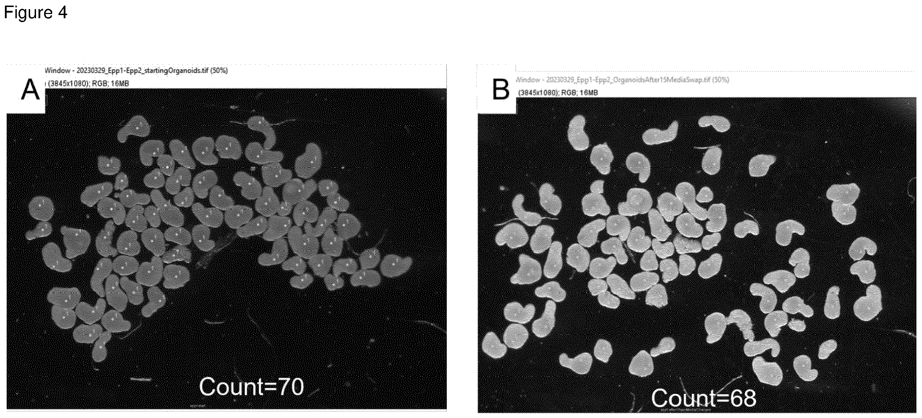

1. 95 3D in-vitro models (fixed embryonic organoids, such as gastruloids) were divided into two sets (one of 70 gastruloids, the other one of 25), imaged, and counted (Fig. 4A).
2. Each set was transferred into a different sample container containing 0.5 ml of media (5x SSCT).
3. For each of the containers, using the engineered pipette tip:
   a. The tip was inserted vertically and in the centre of the container. Aspiration was performed while descending with the tip, following the decrease in liquid level.
   b. The media was aspirated, leaving the samples within a small amount of medium.
   c. The aspirated media was discarded.
   d. 0.5 ml of new media (5x SSCT) was added.
4. The points 3a-c were repeated 15 times, resulting in 15x media exchanges.
5. The content of each sample container was imaged and counted again (Fig. 4B)

### Experimental procedure with conventional pipette tips:

1. 93 3D in-vitro models (fixed gastruloids) were divided into two sets (one of 71 gastruloids, the other one of 22), imaged, and counted.
2. The same above-described procedure was applied, using a conventional 1 ml pipette tip only.
3. After 15x media exchanges, the content of each sample container was imaged and counted again.

### Results:

1. Engineered pipette tip: the final total number of gastruloids after 15x medium exchanges was 94, i.e. 98.95 % of the original ones (one gastruloid got transferred from the first to the second tube).
2. Conventional pipette tip: the final total number of gastruloids after 15x medium exchanges was 49, i.e. 52.69 % of the original ones.
3. See Table 1 (below) for a summary.

### Comparison between conventional and engineered tips:

1. Conventional pipette tip:
   a. The outcome would be always dependent on the experience of the researcher and their attention during each step.
   b. In our lab, where these operations are routinely done, sample loss after multiple media exchanges varies between 10-40% for average-experienced researchers.
   c. The researcher would need to introduce angular tilts between the tip and the container to reduce the generated turbulence, visually checking the movements of the samples, also eventually through a microscope, to actively avoid their aspiration.
   d. After dispensing new media, as the samples move following the liquid turbulence, additional time needs to be accounted for, to let the samples sediment at the bottom of the container.
2. Pipette tip according to the present invention:
   a. During media aspiration, the pipette tip was inserted into the sample container in a vertical direction, without introducing angular tilts.
   b. During aspiration, no particular attention was given to the movements of gastruloids nor to actively avoid their aspiration, to mimic a quasi-blind operation.
   c. During media dispensing, the liquid is by design inserted into the container following the container's walls, reducing the turbulence. This reduces potential damages to the samples and their movement, therefore reducing the time to wait between consecutive steps for their sedimentation.
   d. The operations were performed by a not particularly trained person, who had performed similar operations (multiple media exchanges of floating samples) less than 10 times before the current assay. Therefore, the new design reduces the outcome's dependency to the operator's experience and attention.

### Experiment 2: Automatic media exchange

### Experimental procedure with engineered pipette tips:

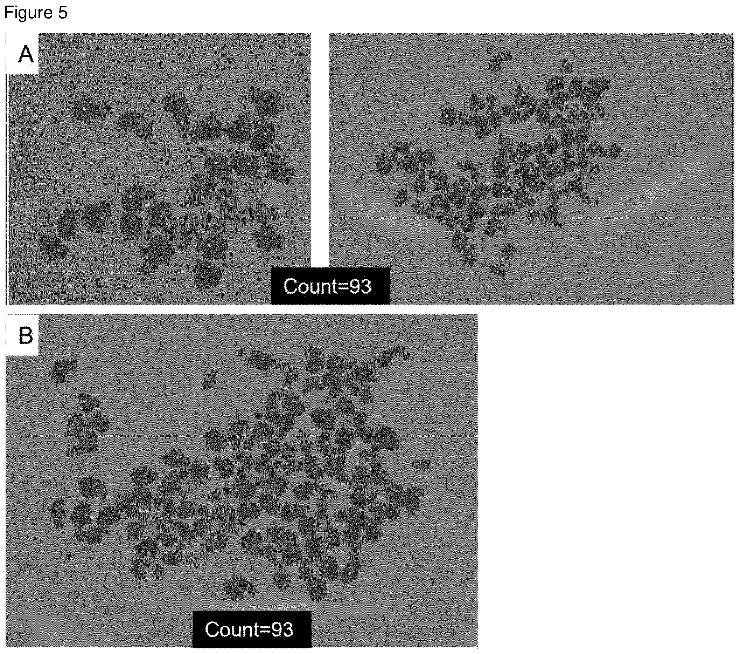

1. 93 3D in-vitro models (fixed gastruloids) were imaged, counted (Fig. 5A), and transferred to a sample container with 0.5 ml of media (5x SSCT).
2. On this container, using the engineered pipette tip and the automated machine:
   a. The tip was inserted vertically and in the centre of the container. Aspiration was performed while descending with the tip, following the decrease in liquid level.
   b. The media was aspirated, leaving the samples within a small volume of media.
   c. The aspirated medium was discarded.
   d. 0.5 ml of new medium (5x SSCT) was added.
3. The points 2a-c were repeated 20 times, resulting in 20x media exchanges.
4. The content of the sample container was imaged and counted again (Fig. 5B).

### Experimental procedure with conventional pipette tips:

1. 92 3D in-vitro models (fixed gastruloids) were imaged and counted.
2. The same above-described procedure was applied, using a conventional 1 ml pipette tip on the automated machine.
3. After 20x media exchanges, the content of the sample container was imaged and counted again.

### Outcomes:

1. Engineered pipette tip: The final total number of gastruloids after 20x media exchanges was 93, i.e. 100 % of the original ones.
2. Conventional pipette tip: The final total number of gastruloids after 20x media exchanges was 58, i.e. 63.01 % of the original ones.
3. See Table 1 for a resume of the comparison.

**Table 1: Summary of the experimental outcomes, comparing the usage of engineered and conventional pipette tips in both manual and automatic media exchange procedures.**

| Method of pipetting | Trial No. | Tip according to the invention | | | Conventional Tip | | |
|---|---|---|---|---|---|---|---|
| | | Before | After | % samples retrieved | Before | After | % samples retrieved |
| Manual | Trial 1 | 70 | 68 | -99% | 68 | 26 | -53% |
| | Trial 2 | 25 | 26 | | 36 | 13 | |
| Robotic (automatic) | Trial 1 | 93 | 93 | 100% | 92 | 58 | ~63% |

Comparison between conventional tips and tips according to the invention:
1. Conventional pipette tip:
   a. The machine is a simple liquid handler, i.e. would not be able to process 3D samples.
   b. Samples are aspirated.
2. Tips according to the invention:
   a. Automated 3D sample handling is enabled.
   b. This transfers all the advantages of automation to this field:
      i. outcomes are not dependent on human factors (attention, experience, etc).
      ii. High-throughput is enabled.
      iii. Researchers can spend more time on the experimental design, outcome interpretation, and less time in tedious tasks.
   c. Over-engineering is avoided (no need for complicated imaging system to guide the pipette tip).
   d. Can convert liquid handlers to "3D sample handlers".

### Reference number list

(p) proximal end
(d) distal end
(l) longitudinal axis
(1) Pipette tip
(2) aperture at the proximal end
(3) aperture at the distal end
(4) plug
(5) ledge or protrusion
(6) channel
(7) common/conventional pipette tip

## Claims

1. A pipette tip (1) for handling of liquids,
comprising a proximal end (p) and a distal end (d) and a longitudinal axis (l) extending therebetween,
an aperture (2) at the proximal end positioned essentially concentric to the longitudinal axis (l) for connecting the tip to a pipette,
wherein the distal end (d) of the tip is closed; and
at least one aperture (3) close to the distal end,
**characterized in that**
the at least one aperture (3) at the distal end (d) is positioned in a way that objects positioned essentially in line with the longitudinal axis are not directly affected and/or impaired by the stream of liquid when handling liquids with the pipette tip.

2. The pipette tip (1) according to claim 1, wherein the at least one aperture at the distal end (3) forms an angle of between 20 and 180 degrees, preferably between 90 and 180 degrees with the longitudinal axis (l) and is preferably positioned in an essentially orthogonal arrangement.

3. The pipette tip (1) according to claim 1 or 2, wherein the distal end (d) of the tip is formed with the distal end (3) being closed, or the distal end is closed by a separate plug (4).

4. The pipette tip (1) according to any one of claims 1 to 3, wherein the distal end (d) of the tip is formed with a ledge or protrusion (5) positioned essentially perpendicular to the longitudinal axis (I), preferably in an essentially round shape, which preferably creates a trap for the sample and/or is provided off-centered from the longitudinal axis (I).

5. The pipette tip (1) according to claim 4, wherein the ledge or protrusion (5) comprises a channel (6) for the flow of the liquids as handled.

6. The pipette tip (1) according to any one of claims 1 to 5, wherein the pipette tip is configured as being ejectable from a pipette tip by an ejection mechanism, for example of a handheld pipette or of a liquid handling workstation.

7. The pipette tip (1) according to any one of claims 1 to 6, wherein the pipette tip is configured as maintaining compatibility with existing micropipettes, and preferably overall is essentially cone-shaped.

8. The pipette tip (1) according to any one of claims 1 to 7, wherein the pipette tip is connected to a micropipette either directly or via an adapter piece, such as a common pipette tip (7).

9. The pipette tip (1) according to any one of claims 1 to 8, wherein the pipette tip is a disposable pipette tip or a reusable pipette tip, such as a washable stainless-steel tip.

10. The pipette tip (1) according to any one of claims 1 to 9, wherein the liquid is selected from a group comprising: a buffer or cell culture medium for cultivating and/or maintaining and/or storing one or more samples, in particular for 3D organoid culture, tissue culture, preserving organs or parts thereof, a reagent for treating the sample, preferably for performing a cell lysis reaction, a staining reaction, a binding reaction, or for removing an embedding medium and/or a transport liquid for collecting particles for further analysis.

11. An assembly, comprising the pipette tip (1) according to any one of claims 1 to 10, attached either directly or via an adapter piece, such as a common pipette tip, to a micropipette, such as a handheld pipette or to a pipette of a liquid handling workstation, such as a positive displacement pipette, in particular an air displacement pipette.

12. A method of treating a sample with a liquid, the method comprising the following steps, i) providing the pipette tip (1) according to any one of claims 1 to 10, ii) attaching the pipette tip (1) either directly or via an adapter piece, such as a common pipette tip, to a micropipette, iii) a) aspirating a liquid into the pipette tip (1), and iv) a) dispensing the liquid from the pipette tip (1) into a suitable culture vessel comprising the sample, or iii) b) aspirating a liquid into the pipette tip (1) from the culture vessel comprising the sample, and iv) b) dispensing the liquid from the pipette tip (1) into a suitable vessel or container, in a way that objects positioned essentially in line with the longitudinal axis are not directly affected and/or impaired by the stream of liquid when handling liquids with the pipette tip (1).

13. The method according to claim 12, wherein the culture vessel is a cell culture vessel, such as a 3D cell culture vessel, and the objects are selected from cells, patient-derived tissues, organs or parts thereof, embryonic cells, embryos and organoids.

14. Use of the pipette tip (1) according to any one of claims 1 to 10 for handling of liquids and/or for treating a sample with a liquid in a way that objects positioned essentially in line with the longitudinal axis are not directly affected and/or impaired by the stream of liquid when handling liquids with the pipette tip (1).

15. A kit for handling of liquids and/or for treating a sample with a liquid, wherein comprising a liquid selected from a group comprising: a buffer or cell culture medium for cultivating and/or maintaining and/or storing one or more samples, in particular for 3D organoid culture, and further comprising the pipette tip (1) according to any one of claims 1 to 10.
